# EUROPEAN PATENT SPECIFICATION

(11) **EP 4 583 767 B1**
(45) Date of publication and mention of the grant of the patent: **07.10.2026**
(21) Application number: 23777030.0
(22) Date of filing: 05.09.2023
(51) Int. Cl.: A61B 5/026, A61B 5/024, A61B 5/02

(54) **DIAGNOSTIC DEVICE FOR ASSESSING THE ENDOTHELIAL FUNCTION OF A HUMAN SUBJECT**
DIAGNOSTISCHE VORRICHTUNG ZUR BEURTEILUNG DER ENDOTHELFUNKTION EINES MENSCHLICHEN SUBJEKTS
DISPOSITIF DE DIAGNOSTIC POUR ÉVALUER LA FONCTION ENDOTHÉLIALE D'UN SUJET HUMAIN

(30) Priority: 06.09.2022 IT 202200018207
(43) Date of publication of application: 16.07.2025
(73) Proprietor: Strumedical S.r.l., 62010 Montecassiano (MC) (IT)
(72) Inventor: CALAMANTI, Chiara, 62010 Montecassiano (MC) (IT); RABUINI, Mario, 62010 Montecassiano (MC) (IT)
(74) Representative: Studio Torta S.p.A.
(86) International application number: PCT/IB2023/058777
(87) International publication number: WO 2024/052815

(56) References cited:
- US-A1- 2007 225 606
- US-A1- 2020 187 789
- LAYLI S GOLDOOZIAN ET AL: "Electrical analog model of arterial compliance during reactive hyperemia", BIOMEDICAL ENGINEERING (MECBME), 2011 1ST MIDDLE EAST CONFERENCE ON, IEEE, 21 February 2011 (2011-02-21), pages 49 - 53, XP031942559, ISBN: 978-1-4244-6998-7, DOI: 10.1109/MECBME.2011.5752062
- BADE GEETANJALI ET AL: "Contribution of systemic vascular reactivity to variability in pulse volume amplitude response during reactive hyperemia", EUROPEAN JOURNAL OF APPLIED PHYSIOLOGY, SPRINGER VERLAG, HEIDELBERG, DE, vol. 119, no. 3, 14 January 2019 (2019-01-14), pages 753 - 760, XP036704951, ISSN: 1439-6319, [retrieved on 20190114], DOI: 10.1007/S00421-018-04066-6

## Description

### CROSS-REFERENCE TO RELATED APPLICATIONS

This Patent Application claims priority from Italian Patent Application No. 102022000018207 filed on September 6, 2022.

### TECHNICAL FIELD

The present invention relates to a diagnostic device for assessing the endothelial function of a human subject.

### BACKGROUND

The endothelium is the largest organ of the human body and covers all the vessels of the cardiovascular system (heart, arteries, veins, capillaries and lymphatic system). Considered as simple tissue in the past, on the contrary the endothelium carries out a role of primary importance in the modulation of the blood vessel tone and of the hematic flow, besides other numerous functions such as the regulation of the inflammatory and immune processes, the vascular permeability, etc.. A damage to the endothelium entails a pathological condition characterized by a reduced vasodilatation, contributing towards various cardiovascular alterations such as atherosclerosis, hypertension and thrombosis.

To date, the most widespread method for assessing the endothelium is measuring, by means of ultrasound examination, the diameter of the brachial artery under basal conditions, subsequently inducing a brief ischemia in a forearm of the subject and then measuring the variations of the diameter in the brachial artery after the ischemia.

The aforementioned method for assessing the endothelium has the advantage of not being invasive, but unfortunately, since it is an ultrasound examination, it strongly depends on the ability of the doctor performing it and thus has a reduced inter- and intra-operator reproducibility.

In order to try increasing the reproducibility, a mechanical arm having a micrometric adjustment so as to prevent the vascular probe from moving and a specific software for measuring the calibre variations in the artery are often utilized. In other words, in the clinical practice the aforementioned method is laborious to perform and therefore not very practicable. Hence, such method is mostly utilized within the scope of clinical investigation, i.e. does not fall within the daily hospital practice, let alone in the outpatient practice.

A paper by Layli S. Goldoozian et al., titled "Electrical Analog Model of Arterial Compliance during Reactive Hyperemia", published by 2011 1st Middle East Conference un Biomedical Engineering (MECBME 201), IEEE, on 21 February 2011, discloses a low skilled-operator demanding method for assessing the endothelial function, based on the photoplethysmogram (PPG) changes during reactive hyperemia. According to that method, the PPG changes following reactive hyperaemia are modelled using time-varying passive electrical elements (R, L and C). The output of the electric analog model matches relatively well the PPG-AC signal.

U.S. Patent Application Publication US2020/0187789A1 discloses method and apparatus to determine the vascular reactivity based on a measured response of the vasculature to reactive hyperemia utilizing continuous skin monitoring of inherent temperature on a digit distal (downstream) to an occluded arterial flow. The apparatus comprises an inflatable cuff for placement around a subject's limb, a digital thermal measuring device and photoplethysmography measuring device for placement on a finger of the subject's limb wearing the cuff and a second digital monitoring device and photoplethysmography measuring device for placement on a finger of the subject's contralateral limb.

### SUMMARY

The object of the present invention is to provide an instrument which allows assessing the endothelial function of a human subject, which is exempt from the above-described drawbacks and, at the same time, is cost-effective to manufacture, easy to utilize and especially provides results substantially independent of the operator using it.

In accordance with the present invention, a diagnostic device for assessing the endothelial function of a subject is provided, in accordance with the appended claims.

### BRIEF DESCRIPTION OF THE DRAWINGS

The present invention will now be described with reference to the accompanying drawings, which illustrate a non-limiting example embodiment thereof, wherein:
- Figure 1 illustrates the diagnostic device of the present invention for assessing the endothelial function of a human subject;
- Figure 2 illustrates the diagnostic device of Figure 1 when in use on the human subject;
- Figure 3 illustrates a block diagram of internal parts of the diagnostic device of Figure 1;
- Figure 4 illustrates a simplified block diagram of a part of the software architecture of a unit of the diagram of Figure 3;
- Figure 5 illustrates a simplified flowchart relative to a measuring cycle executed by the diagnostic device of Figure 1;
- Figure 6 illustrates an example of the two filtered signals derived from the signals acquired by two sensors of the diagnostic device of Figure 1 during the measuring cycle of Figure 5, with reference to the calculation of an endothelial function index;
- Figures 7A and 7B illustrate two examples of one of the two filtered signals of Figure 6 obtained from a healthy human subject and from a subject having an endothelial dysfunction, respectively; and
- Figure 8 illustrates another example of one of the two filtered signals of Figure 6 with reference to the calculation of a further endothelial function index; and
- Figure 9 illustrates a waveform of a heartbeat part of one of the two filtered signals of Figure 6, on which some points of reference are illustrated.

### DESCRIPTION OF EMBODIMENTS

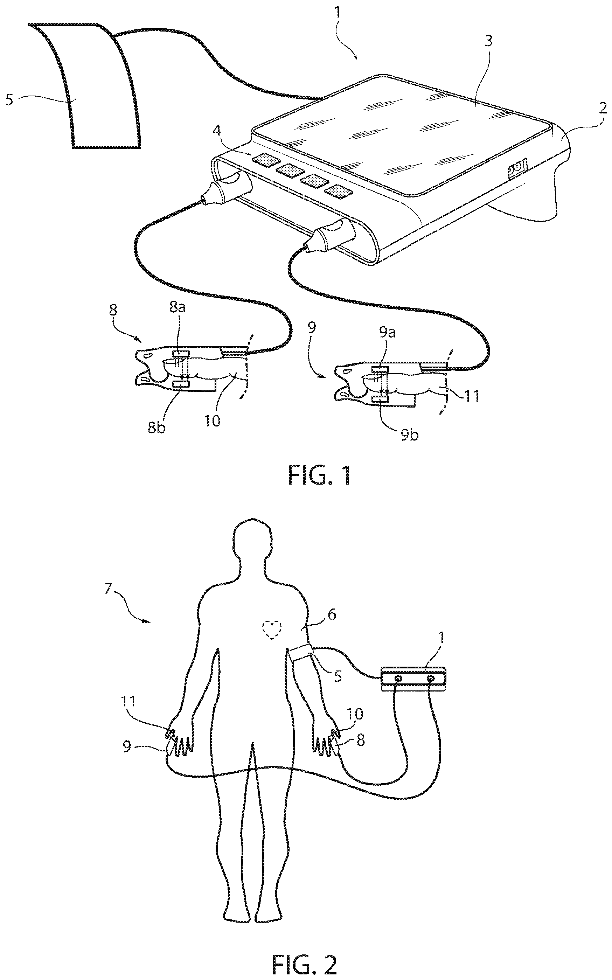

In Figures 1 and 2, reference numeral 1 generically indicates, as a whole, the diagnostic device of the present invention. The diagnostic device 1 comprises a box-shaped body 2, a display 3, preferably of touch-screen type, facing from an upper side of the box-shaped body 2, a push-button interface 4 for enabling an operator to input basic commands, a sphygmomanometric sleeve 5 applicable to a forearm 6 (Figure 2) of a subject 7 and two photoplethysmographic sensors 8 and 9 applicable to two respective fingers of two different hands of the subject 7. In particular, as is illustrated in Figure 2, the photoplethysmographic sensor 8 is applied to a finger 10, for example the index finger, of the hand of the forearm 6 and the other photoplethysmographic sensor 9 is applied to a finger 11, for example the index finger, of the other hand of the subject 7.

Each photoplethysmographic sensor 8, 9 comprises a source of light or infrared radiation 8a, 9a adapted to light a portion of skin of the finger 10, 11 and a receiver 8b, 8b which detects the variations of light absorbed by the portion of skin, such variations being due to the blood perfusion in the dermis and in the subcutaneous regions of the portion of skin. The photoplethysmographic sensors 8 and 9 are of known type and thus will not be described in further detail.

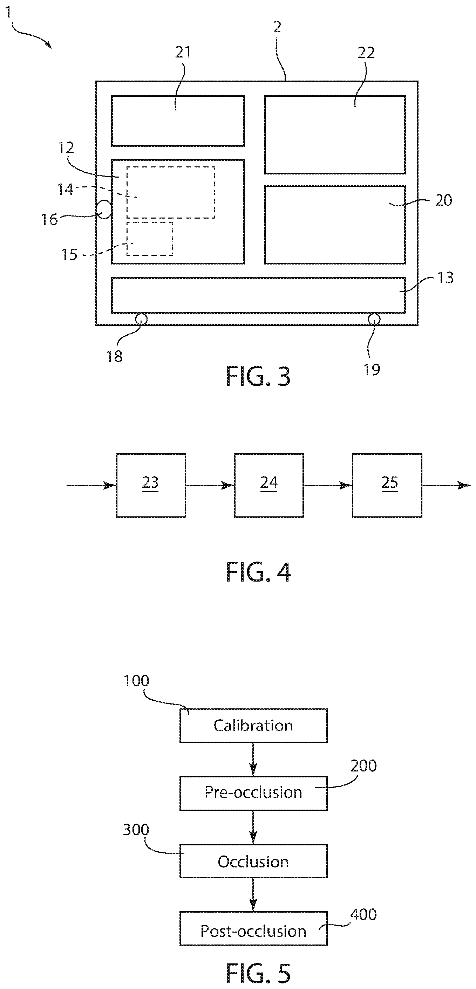

With reference to Figure 3, the diagnostic device 1 comprises, inside the box-shaped body 2, a pressure adjustment unit 12 for adjusting the pressure of the sphygmomanometric sleeve 5 and a measuring unit 13 for controlling the pressure adjustment unit 12 and the photoplethysmographic sensors 8 and 9 and for acquiring, by means of the latter, a first signal S1 relative to the finger 10 and a second signal S2 relative to the other finger 11. The pressure adjustment unit 12 comprises a pump 14, an outlet valve 15, and a door 16 for the pneumatic connection of the sphygmomanometric sleeve 5. The measuring unit 13 comprises two doors 18 and 19 for the electrical connection of the photoplethysmographic sensors 8 and 9.

The diagnostic device 1 comprises, still inside the box-shaped body 2, a processing and control unit 20, a battery pack 21 for providing electric energy, and a power supply management unit 22, which manages the power supply of all the electrical and electronic parts of the diagnostic device 1.

The processing and control unit 20 is configured to control the measuring unit 13 so as to execute, in use, a measuring cycle which leads to the determination of one or more endothelial function indexes and, optionally, of heartbeat parameters which can be utilized for studying the severity of a possible endothelial dysfunction, as it will be specifically explained in the following of the present document. The measuring cycle is executed in an automatic manner.

With reference to Figure 4, the processing and control unit 20 comprises at least one pre-processing module, and in particular three pre-processing modules indicated by 23, 24 and 25, for filtering the two signals S1 and S2 so as to obtain two respective filtered signals SF1 and SF2. The processing and control unit 20 is configured to determine the aforementioned endothelial function indexes and heartbeat parameters on the basis of the filtered signals SF1 and SF2.

Specifically, said three pre-processing modules comprise in cascade, in the following order, a low-pass filter 23, an artefact removal module 24 and an anomalous values removal module 25. The modules 23, 24 and 25 process in an identical and mutually independent manner the two signals S1 and S2. In other words, the functions of the modules 23, 24 and 25 are duplicated for the two signals S1 and S2.

The low-pass filter 23 is adapted to remove the oscillations at high-frequencies, with respect to those of the heartbeat, generated by ambient electromagnetic disturbances, such as for example the electromagnetic field generated by the electric network which comprises a fundamental frequency at 50 Hz and harmonic oscillations at higher frequencies.

The artefact removal module 24 is adapted to remove a signal component due to the body movements of the subject 7, and in particular of the fingers 10 and 11 with respect to the photoplethysmographic sensors 8 and 9. For example, the artefact removal module 24 implements a procedure known in the electrocardiographic field as baseline wander filtering.

The anomalous values removal module 25 is adapted to remove the isolated peak values of the signal, which are also often due to the body movements of the subject. For example, the anomalous values removal module 25 implements a procedure similar to the one followed by the "isoulier" function by The MathWorks Inc.

The three pre-processing modules 23, 24 and 25 are implemented in hardware, or are implemented by a software. In the latter case, the software is stored in a memory of the processing and control unit 20 and is executed by a processor of the processing and control unit 20.

With reference to Figure 5, the measuring cycle, which is controlled by the processing and control unit 20 so as to be executed automatically, comprises, in the following order, a calibration phase (step 100), in which the photoplethysmographic sensors 8 and 9 are calibrated on the fingers 10 and 11 and the sphygmomanometric sleeve 5 is left deflated, a basal or pre-occlusion phase (step 200), in which the signals S1 and S2 are acquired while the sphygmomanometric sleeve 5 is left deflated, an occlusion phase (step 300), in which the signals S1 and S2 are acquired and the sphygmomanometric sleeve 5 is rapidly inflated, kept inflated at a maximum pressure value Pmax such to occlude the brachial artery of the forearm 6 and determined according to the signal S1, and then rapidly deflated, and a post-occlusion phase (step 400), in which the signals S1 and S2 are acquired while the sphygmomanometric sleeve 5 is left deflated. In the following, for simplicity, the phases 200, 300 and 400 are also called as a whole measurement phases.

With regard to the calibration phase, the processing and control unit 20 is configured to control the measuring unit 13 so that the latter measures, on the basis of the signals S1 and S2, a perfusion index for each of the photoplethysmographic sensors 8 and 9 and verifies that it is greater than a threshold value. The measurement of the perfusion index is known. Furthermore, the measuring unit 13 adjusts the intensity of the sources 8a and 9a until the receivers 8b and 9b detect respective light signals above a certain light threshold value. This enables adapting the operation of the photoplethysmographic sensors 8 and 9 to the characteristics of the tissue of the fingers 10 and 11 and to the ambient light.

At the beginning of the occlusion phase, the sphygmomanometric sleeve 5 is rapidly inflated to a pressure value P1 corresponding to a substantially null value of the signal S1 and at the end of the occlusion phase the sphygmomanometric sleeve 5 is rapidly deflated. When the signal S1 is close to the null value, it means that the blood flow in the finger 10 is substantially null, and thus that the brachial artery of the forearm 6 is actually occluded. The maximum pressure value Pmax is determined according to the pressure value P1.

Advantageously, the maximum pressure value Pmax is given by the sum of the pressure value P1 with a predetermined pressure increase ΔP. This allows preventing the pressure from not being sufficient for occluding the brachial artery due to muscle spasms or movements of the forearm 6. For example, the pressure increase ΔP is between 30 and 50 mmHg, preferably it is equal to 40 mmHg. The temporary occlusion of the brachial artery induces a temporary ischemia at the level of the forearm 6.

The calibration phase has a duration T1 between 1 and 5 minutes, preferably equal to 3 minutes. The pre-occlusion phase, the occlusion phase and the post-occlusion phase have respective durations T2, T3, T4, each between 3 and 6 minutes, preferably equal to 5 minutes.

The durations T1, T2, T3, T4 are singularly pre-settable by the operator, depending on the investigations which have to be carried out. In accordance with an embodiment, the duration T1 is equal to 3 minutes and the durations T2, T3 and T4 are all three equal to 5 minutes. In accordance with a further embodiment, the duration T1 is equal to 3 minutes, the duration T2 is equal to 3 minutes, the duration T3 is equal to 4 minutes and the duration T4 is equal to 3 minutes.

The inflation of the sphygmomanometric sleeve 5 to the maximum pressure value Pmax is an initial transitory sub-phase of the occlusion phase which has a duration between 30 and 40 seconds.

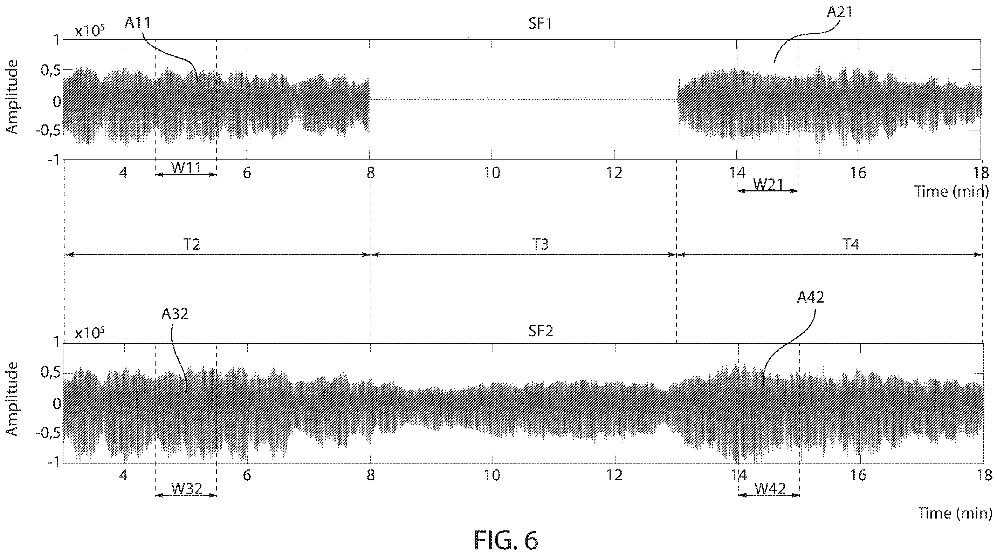

With reference to Figure 6, which illustrates an example of the filtered signals SF1 and SF2 during the measurement phases 200, 300, 400, the processing and control unit 20 is configured to determine a first endothelial function index, indicated by EI, in the following manner.

Four areas subtended by the envelopes of the filtered signals SF1 and SF2 within four respective time windows are calculated. In particular, these four areas are:
- a first area A11 subtended by the envelope of the filtered signal SF1 within a first time window W11 of the pre-occlusion phase, the latter extending according to a time interval having the duration T2;
- a second area A21 subtended by the envelope of the filtered signal SF1 within a second time window W21 of the post-occlusion phase, the latter extending according to a time interval having the duration T4;

- a third area A32 subtended by the envelope of the filtered signal SF2 within a third time window W32 of the pre-occlusion phase; and
- a fourth area A42 subtended by the envelope of the filtered signal SF2 within a fourth time window W42 of the post-occlusion phase.

The endothelial function index EI is calculated as the product of a ratio of the area A21 to the area A11 by a ratio of the area A32 to the area A42, i.e. with the following formula: $IE = \frac{A 21}{A 11} ⋅ \frac{A 32}{A 42} .$

The time windows W11, W21, W32 and W42 have respective durations, each between 0.5 and 1.5 minutes, preferably equal to 1 minute. The durations of the windows W11, W21, W32 and W42 can be set at the discretion of the operator. The location of the time windows W11 and W32 within the pre-occlusion phase, i.e. within the interval having duration T2, and of the time window W42 within the post-occlusion phase, **i.e.** within the interval having duration T4, is not relevant. The time window W21 begins after a predetermined delay D from the end of the occlusion phase, **i.e.** at the end of the duration T3. The delay D is between 0.5 and **1.5** minutes, preferably equal to 1 minute. However, also the precise location of the windows W11, W21, W32 and W42 within the relative pre-occlusion and post-occlusion phases is settable at the discretion of the operator.

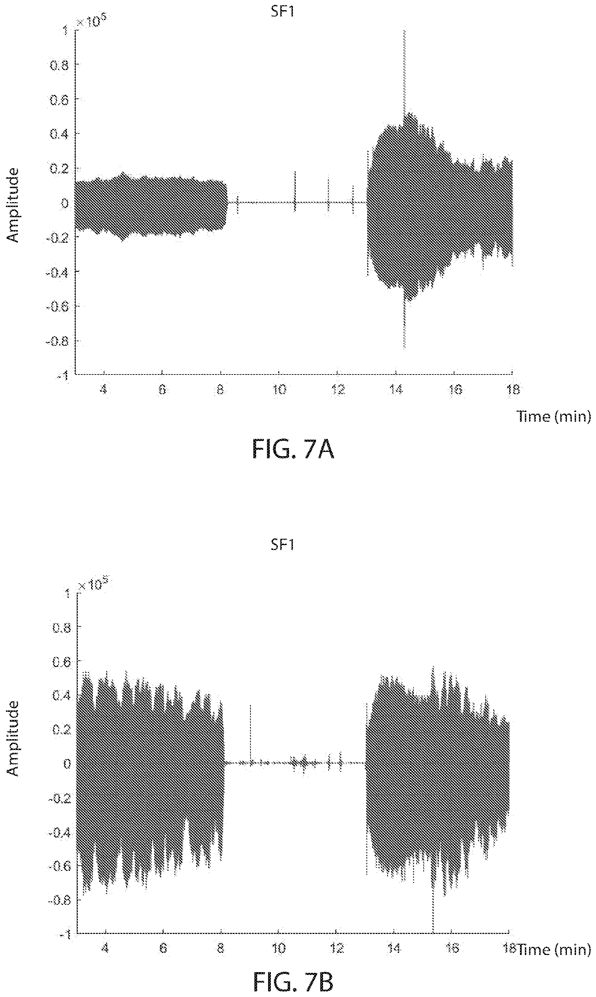

The endothelial function index EI was assessed with a clinical investigation, which highlighted the existence of a threshold value which allows providing indications on the state of the subject 7. Figure 7A illustrates the course of the filtered signal SF1 of a healthy subject, to which an endothelial function index EI greater than said threshold value corresponds, and Figure 7B illustrates the course of the filtered signal SF1 of a subject having an endothelial dysfunction, to which an endothelial function index EI lesser than said threshold value corresponds.

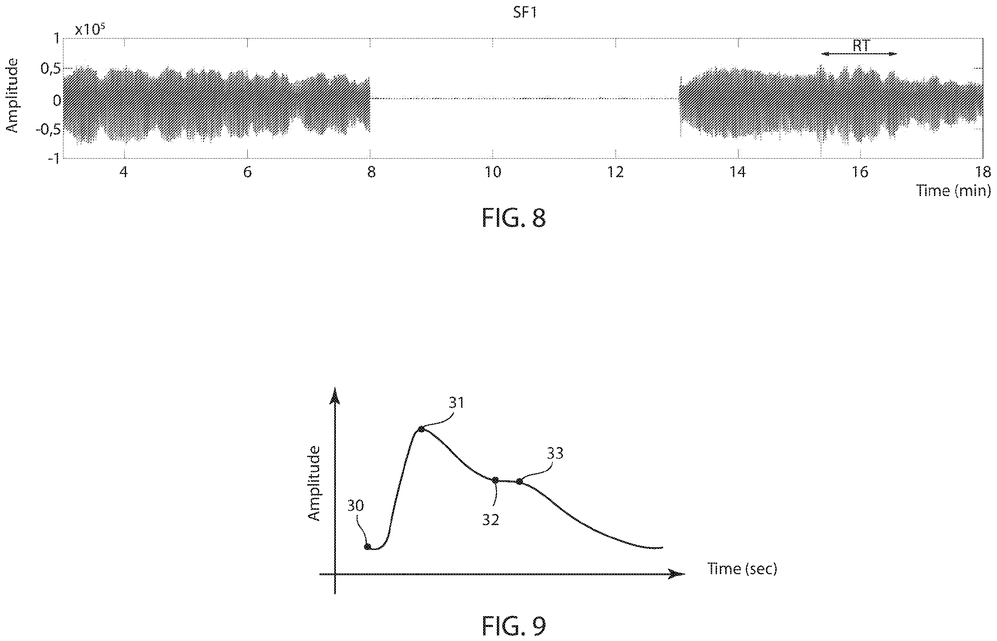

With reference to Figure 8, which illustrates another example of the filtered signal SF1 during the measurement phases 200, 300, 400, the processing and control unit 20 is configured to determine a further endothelial function index, indicated by RT and called in the following recovery time.

An average amplitude of the filtered signal SF1 is calculated during the post-occlusion phase. The recovery time RT is calculated as that time interval in the post-occlusion phase between the instant in which the filtered signal SF1 reaches the maximum amplitude and the instant in which the filtered signal SF1 reaches said average amplitude.

The higher the recovery time RT, the greater the endothelial dysfunction. This can be explained by the fact that a high value of recovery time RT expresses a poor recovery capacity of the endothelium following the temporary ischemia caused by the occlusion phase, and the poor recovery capacity is necessarily linked to a reduced endothelial function. The combined reading of the value of the recovery time RT and of the endothelial function index could provide an indication of how serious a pathology linked to the endothelial dysfunction is.

The numerical values of the endothelial function index EI and of the recovery time RT can be displayed directly on the display 3 of the diagnostic device 1.

Optionally, the processing and control unit 20 is configured to process the filtered signal SF1 in the pre-occlusion phase or/and in the post-occlusion phase so as to extract one or more heartbeat parameters in the manner described in the following.

The filtered signal SF1 is composed of a sequence of a high number of heartbeat waveforms, each of which is similar to the example illustrated in Figure 9. With reference to Figure 9, each heartbeat waveform is characterized by points of reference, known in the electrocardiographic and photopletysmographic fields, such as for example the dicrotic minimum 30, the systolic peak 31, the dicrotic notch 32 and the diastolic peak 33, and by heartbeat parameters also known in the electrocardiographic field and calculable on the basis of the aforementioned points of reference of one waveform or of two contiguous waveforms. In particular, the aforementioned heartbeat parameters are known in the literature as:
- Systolic Amplitude,
- Inflection Point Area,
- Pulse Interval,
- Heart Rate,
- Peak-to-Peak Time,
- Stiffness Index,
- Augmentation Index.

The heartbeat parameters which are determined by the processing and control unit 20 are thus selected from a group comprising Systolic Amplitude, Inflection Point Area, Pulse Interval, Heart Rate, Peak-to-Peak Time, Stiffness Index, Augmentation Index.

However, the aforementioned heartbeat parameters are defined on a single heartbeat waveform.

With the purpose of associating at least one heartbeat parameter with the filtered signal SF1, from the latter a predetermined number of signal portions is selected corresponding to the waveforms of as many random heartbeats, in the pre-occlusion phase or/and in the post-occlusion phase. In other words, the signal portions corresponding to as many heartbeats are randomly selected along the overall waveform of the filtered signal SF1 in the pre-occlusion phase or/and in the post-occlusion phase. For each of the selected heartbeats, a value of at least one heartbeat parameter is calculated on the basis of the waveform of the heartbeat and then an average value of said heartbeat parameter is calculated among all the values of the selected heartbeats.

In particular, for each selected heartbeat, the calculation of the value of the heartbeat parameter is preceded by the identification of the points of reference 30-33 of the waveform of the heartbeat. The points of reference 30-33 are identified by processing first derivative and second derivative of the waveform of the heartbeat. The value of the heartbeat parameter is calculated on the basis of the points of reference 30-33 of the relative waveform by means of simple mathematical relationships. For example, the systolic amplitude is equal to the difference of the amplitude values of the systolic peak 31 and of the dicrotic minimum 30 and the peak-to-peak time is equal to the difference of the time instants of the systolic peak 31 and of the diastolic peak 33.

The above-described measuring cycle, with reference to Figure 5, and the calculation of the endothelial function indexes EI, RT and of the heartbeat parameters on the basis of the filtered signals SF1 and SF2 are implemented by a software which is stored in the memory of the processing and control unit 20 and is executed by the processor of the processing and control unit 20.

The heartbeat parameters allow refining a diagnosis based on the two endothelial function indexes EI and RT. Advantageously, the processing and control unit 20 is configured to execute a machine learning algorithm which allows classifying the severity of an endothelial dysfunction in correlation with one or more heartbeat parameters.

The main advantage of the above-described diagnostic device 1 is to enable a diagnosis of the endothelium in an automatic manner and without the need for the intervention of a specialized doctor in the utilization of complex medical instruments. The diagnosis can be synthetically expressed on the display by means of numerical values of one or more suitably calculated endothelial function indexes. Furthermore, the diagnostic device 1 is relatively cost-effective to manufacture, as it comprises widespread components, such as the sphygmomanometric sleeve 5, a pressure adjustment unit 12 comprising a pump 14 and an outlet valve 15, and two photoplethysmographic sensors 8 and 9.

## Claims

1. A diagnostic device for assessing the endothelial function of a subject (7), the diagnostic device (1) comprising a sphygmomanometric sleeve (5) applicable to a first forearm (6) of the subject (7), a pressure adjustment unit (12) for adjusting the pressure of the sphygmomanometric sleeve (5), two photoplethysmographic sensors (8, 9) applicable to a first finger (10) of the hand associated with the first forearm (6) and, respectively, to a second finger (11) of the other hand of the subject (7), a measuring unit (13) for controlling the pressure adjustment unit (12) and the photoplethysmographic sensors (8, 9) and for acquiring, by means of the latter, a first signal (S1) relative to the first finger (10) and a second signal (S2) relative to the second finger (11), and a processing and control unit (20), which is configured to control the measuring unit (13) so as to execute, in use, a cycle comprising, in the following order, a first phase (100), in which the photoplethysmographic sensors (8, 9) are calibrated on the first finger (10) and second finger (11), a second phase (200), in which the sphygmomanometric sleeve (5) is left deflated, a third phase (300), in which the sphygmomanometric sleeve (5) is kept inflated to a maximum pressure value (Pmax) determined according to the first signal (S1), and a fourth phase (400), in which the sphygmomanometric sleeve (5) is left deflated; the processing and control unit (20) comprising at least one pre-processing module (23-25) for filtering first and second signals (S1, S2) to obtain a first and second filtered signal (SF1, SF2) and being configured to determine at least one endothelial function index (EI, RT) on the basis of at least the first filtered signal (SF1) in the second phase (200) and the fourth phase (400).

2. The diagnostic device according to claim 1, wherein the processing and control unit (20) is configured to control the measuring unit (13) such that at the beginning of the third phase (300) the sphygmomanometric sleeve (5) is rapidly inflated to a first pressure value (P1) corresponding to a substantially null value of the first signal (S1) and at the end of the third phase (300) the sphygmomanometric sleeve (5) is rapidly deflated; the maximum pressure value (Pmax) being determined as a function of the first pressure value (P1).

3. The diagnostic device according to claim 1 or 2, wherein said first phase (100) has a duration (T1) between 1 and 5 minutes, preferably equal to 3 minutes.

4. The diagnostic device according to any one of claims 1 to 3, wherein said second phase (200), third phase (300) and fourth phase (400) have respective durations (T2-T4), each between 3 and 6 minutes, preferably equal to 5 minutes.

5. The diagnostic device according to any one of claims 1 to 4, wherein said processing and control unit (20) is configured to calculate a first area (A11) and a second area (A21) subtended by the envelope of the first filtered signal (SF1) within a first time window (W11) of the second phase (200) and, respectively, a second time window (W21) of the fourth phase (400), a third area (A32) and a fourth area (A42) subtended by the envelope of the second filtered signal (SF2) within a third time window (W32) of the second phase (200) and, respectively, a fourth time window (W42) of the fourth phase (400), and a first endothelial function index (EI) as the product of a ratio of second area (A21) to first area (A11) by a ratio of third area (A32) to fourth area (A42).

6. The diagnostic device according to claim 5, wherein said first, second, third and fourth time windows (W11, W21, W32, W42) have respective durations, each between 0.5 and 1.5 minutes, preferably equal to 1 minute.

7. The diagnostic device according to claim 5 or 6, wherein said second time window (W21) begins after a predetermined delay (D) from the end of the third phase (300), said delay (D) being between 0.5 and 1.5 minutes, preferably equal to 1 minute.

8. The diagnostic device according to any one of claims 1 to 7, wherein said processing and control unit (20) is configured to calculate an average amplitude of the first filtered signal (SF1) during the second phase (200) and a second endothelial function index (RT) as that time interval in the fourth phase (400) between the instant in which the first filtered signal (SF1) reaches the maximum amplitude and the instant in which the first filtered signal (SF1) reaches said average amplitude.

9. The diagnostic device according to any one of claims 1 to 8, wherein the processing and control unit (20) is configured to select, from the first filtered signal (SF1) in the second phase (200) or/and in the fourth phase (400), a predetermined number of signal portions corresponding to the waveforms of as many random heartbeats, to calculate, for each of said heartbeats, a value of at least one heartbeat parameter on the basis of its waveform, and to calculate the average value of said heartbeat parameter among all the values of said heartbeats.

10. The diagnostic device according to any one of claims 1 to 9, wherein each of the photoplethysmographic sensors (8, 9) comprises a source of light or infrared radiation (8a, 9a) and a corresponding receiver (8b, 9b) and the processing and control unit (20) is configured to control the measuring unit (13) such that the latter, during said first phase (100), measures and verifies the amplitude of a perfusion index for both photoplethysmographic sensors (8, 9) on the basis of the first and second signals (S1, S2), and adjusts the intensity of the radiation emitted by the radiation sources (8a, 9a) to adapt the operation of the photoplethysmographic sensors (8, 9) to the characteristics of the finger tissue and the ambient light.

11. The diagnostic device according to any one of claims 1 to 10, wherein said at least one pre-processing module (23-25) comprises a low-pass filter (23) and at least an artefact removal module (24), the latter to remove artefacts on the first and second signals (S1, S2) due to movements of the first and second fingers (10, 11) with respect to the photoplethysmographic sensors (8, 9).

## Patentansprüche

1. Diagnosevorrichtung zur Beurteilung der Endothelfunktion eines Patienten (7), wobei die Diagnosevorrichtung (1) eine Blutdruckmanschette (5), die an einem ersten Unterarm (6) des Patienten (7) anlegbar ist, eine Druckeinstellungseinheit (12) der Blutdruckmanschette (5), zwei photoplethysmographische Sensoren (8, 9), die an einem ersten Finger (10) der mit dem ersten Unterarm (6) verbundenen Hand bzw. an einem zweiten Finger (11) der anderen Hand des Patienten (7) anlegbar sind, eine Messeinheit (13) zur Steuerung der Druckeinstellungseinheit (12) und der photoplethysmographischen Sensoren (8, 9) sowie zur Erfassung eines ersten Signals (S1) in Bezug auf den ersten Finger (10) und eines zweiten Signals (S2) in Bezug auf den zweiten Finger (11) mittels dieser Sensoren, und eine Verarbeitungs- und Steuereinheit (20) umfasst, die konfiguriert ist, um die Messeinheit (13) so zu steuern, dass im Gebrauch ein Zyklus ausgeführt wird, der in der folgenden Reihenfolge eine erste Phase (100) umfasst, in der die photoplethysmographischen Sensoren (8, 9) am ersten Finger (10) und am zweiten Finger (11) kalibriert werden, eine zweite Phase (200), in der die Blutdruckmanschette (5) nicht aufgeblasen bleibt, eine dritte Phase (300), in der die Blutdruckmanschette (5) auf einen gemäß dem ersten Signal (S1) bestimmten maximalen Druckwert (Pmax) aufgeblasen gehalten wird, und eine vierte Phase (400), in der die Blutdruckmanschette (5) nicht aufgeblasen bleibt; wobei die Verarbeitungs- und Steuereinheit (20) mindestens ein Vorverarbeitungsmodul (23-25) zum Filtern des ersten und des zweiten Signals (S1, S2) umfasst, um ein erstes und ein zweites gefiltertes Signal (SF1, SF2) zu erhalten, und so konfiguriert ist, dass sie auf der Grundlage mindestens des ersten gefilterten Signals (SF1) in der zweiten Phase (200) und der vierten Phase (400) mindestens einen Endothelfunktionsindex (EI, RT) bestimmt.

2. Diagnosevorrichtung nach Anspruch 1, wobei die Verarbeitungs- und Steuereinheit (20) so konfiguriert ist, dass sie die Messeinheit (13) so steuert, dass zu Beginn der dritten Phase (300) die Blutdruckmanschette (5) schnell auf einen ersten Druckwert (P1) aufgeblasen wird, der einem im Wesentlichen Nullwert des ersten Signals (S1) entspricht, und am Ende der dritten Phase (300) die Blutdruckmanschette (5) schnell abgeblasen wird; wobei der maximale Druckwert (Pmax) in Abhängigkeit vom ersten Druckwert (P1) bestimmt wird.

3. Diagnosevorrichtung nach Anspruch 1 oder 2, wobei die erste Phase (100) eine Dauer (T1) zwischen 1 und 5 Minuten aufweist, vorzugsweise 3 Minuten beträgt.

4. Diagnosevorrichtung nach einem der Ansprüche 1 bis 3, wobei die zweite Phase (200), die dritte Phase (300) und die vierte Phase (400) jeweilige Dauer (T2-T4) aufweisen, die jeweils zwischen 3 und 6 Minuten, vorzugsweise 5 Minuten, betragen.

5. Diagnosevorrichtung nach einem der Ansprüche 1 bis 4, wobei die Verarbeitungs- und Steuereinheit (20) so konfiguriert ist, dass sie eine erste Fläche (A11) und eine zweite Fläche (A21) berechnet, die von der Hüllkurve des ersten gefilterten Signals (SF1) innerhalb eines ersten Zeitfensters (W11) der zweiten Phase (200) bzw. eines zweiten Zeitfensters (W21) der vierten Phase (400) eine dritte Fläche (A32) und eine vierte Fläche (A42), die von der Hüllkurve des zweiten gefilterten Signals (SF2) innerhalb eines dritten Zeitfensters (W32) der zweiten Phase (200) bzw. eines vierten Zeitfensters (W42) der vierten Phase (400) begrenzt werden, sowie einen ersten Endothelfunktionsindex (EI) als Produkt aus dem Verhältnis der zweiten Fläche (A21) zur ersten Fläche (A11) und dem Verhältnis der dritten Fläche (A32) zur vierten Fläche (A42).

6. Diagnosevorrichtung nach Anspruch 5, wobei das erste, zweite, dritte und vierte Zeitfenster (W11, W21, W32, W42) eine jeweilige Dauer aufweisen, die jeweils zwischen 0,5 und 1,5 Minuten liegen, vorzugsweise 1 Minute betragen.

7. Diagnosevorrichtung nach Anspruch 5 oder 6, wobei das zweite Zeitfenster (W21) nach einer vorbestimmten Verzögerung (D) ab dem Ende der dritten Phase (300) beginnt, wobei die Verzögerung (D) zwischen 0,5 und 1,5 Minuten, vorzugsweise 1 Minute, beträgt.

8. Diagnosevorrichtung nach einem der Ansprüche 1 bis 7, wobei die Verarbeitungs- und Steuereinheit (20) so konfiguriert ist, dass sie eine durchschnittliche Amplitude des ersten gefilterten Signals (SF1) während der zweiten Phase (200) sowie einen zweiten Endothelfunktionsindex (RT) als das Zeitintervall in der vierten Phase (400) zwischen dem Zeitpunkt, zu dem das erste gefilterte Signal (SF1) die maximale Amplitude erreicht, und dem Zeitpunkt, zu dem das erste gefilterte Signal (SF1) die durchschnittliche Amplitude erreicht, berechnet.

9. Diagnosevorrichtung nach einem der Ansprüche 1 bis 8, wobei die Verarbeitungs- und Steuereinheit (20) so konfiguriert ist, dass sie aus dem ersten gefilterten Signal (SF1) in der zweiten Phase (200) und/oder in der vierten Phase (400) eine vorbestimmte Anzahl von Signalabschnitten auswählt, die den Wellenformen ebenso vieler zufälliger Herzschläge entsprechen, für jeden dieser Herzschläge einen Wert mindestens eines Herzschlagparameters auf der Grundlage seiner Wellenform zu berechnen und den Durchschnittswert des Herzschlagparameters aus allen Werten dieser Herzschläge berechnen.

10. Diagnosevorrichtung nach einem der Ansprüche 1 bis 9, wobei jeder der photoplethysmographischen Sensoren (8, 9) eine Licht- oder Infrarotstrahlungsquelle (8a, 9a) und einen entsprechenden Empfänger (8b, 9b) umfasst und die Verarbeitungs- und Steuereinheit (20) so konfiguriert ist, dass sie die Messeinheit (13) derart steuert, dass diese während der ersten Phase (100) die Amplitude eines Perfusionsindexes für beide photoplethysmographischen Sensoren (8, 9) auf der Grundlage des ersten und des zweiten Signals (S1, S2) misst und überprüft und die Intensität der von den Strahlungsquellen (8a, 9a) emittierten Strahlung einstellt, um den Betrieb der photoplethysmographischen Sensoren (8, 9) an die Merkmale des Fingergewebes und des Umgebungslichts anzupassen.

11. Diagnosevorrichtung nach einem der Ansprüche 1 bis 10, wobei das mindestens eine Vorverarbeitungsmodul (23-25) einen Tiefpassfilter (23) und mindestens ein Artefaktentfernungsmodul (24) umfasst, wobei letzteres dazu dient, Artefakte auf dem ersten und dem zweiten Signal (S1, S2) zu entfernen, die auf Bewegungen des ersten und des zweiten Fingers (10, 11) im Hinblick auf die photoplethysmographischen Sensoren (8, 9) zurückzuführen sind.

## Revendications

1. Dispositif de diagnostic destiné à évaluer la fonction endothéliale d'un sujet (7), le dispositif de diagnostic (1) comprenant un brassard tensiométrique (5) pouvant être appliqué sur un premier avant-bras (6) du sujet (7), une unité de réglage de pression (12) destinée à régler la pression du brassard tensiométrique (5), deux capteurs photopléthysmographiques (8, 9) pouvant être appliqués sur un premier doigt (10) de la main associée au premier avant-bras (6) et, respectivement, sur un second doigt (11) de l'autre main du sujet (7), une unité de mesure (13) destinée à commander l'unité de réglage de pression (12) et les capteurs photopléthysmographiques (8, 9) et pour acquérir, au moyen de ces derniers, un premier signal (S1) relatif au premier doigt (10) et un second signal (S2) relatif au second doigt (11), et une unité de traitement et de commande (20), qui est configurée pour commander l'unité de mesure (13) de manière à exécuter, en fonctionnement, un cycle comprenant, dans l'ordre suivant, une première phase (100), au cours de laquelle les capteurs photopléthysmographiques (8, 9) sont étalonnés sur le premier doigt (10) et le second doigt (11), une deuxième phase (200), au cours de laquelle le brassard tensiométrique (5) est laissé dégonflé, une troisième phase (300), au cours de laquelle le brassard tensiométrique (5) est maintenu gonflé à une valeur de pression maximale (Pmax) déterminée en fonction du premier signal (S1), et une quatrième phase (400), au cours de laquelle le brassard tensiométrique (5) est laissé dégonflé ; l'unité de traitement et de commande (20) comprenant au moins un module de prétraitement (23-25) destiné à filtrer les premier et second signaux (S1, S2) afin d'obtenir un premier et un second signal filtré (SF1, SF2) et étant configurée pour déterminer au moins un indice de fonction endothéliale (EI, RT) sur la base d'au moins le premier signal filtré (SF1) au cours de la deuxième phase (200) et de la quatrième phase (400).

2. Dispositif de diagnostic selon la revendication 1, dans lequel l'unité de traitement et de commande (20) est configurée pour commander l'unité de mesure (13) de telle sorte qu'au début de la troisième phase (300), le brassard tensiométrique (5) soit gonflé rapidement jusqu'à une première valeur de pression (P1) correspondant à une valeur sensiblement nulle du premier signal (S1) et qu'à la fin de la troisième phase (300), le brassard tensiométrique (5) soit rapidement dégonflé ; la valeur de pression maximale (Pmax) étant déterminée en fonction de la première valeur de pression (P1).

3. Dispositif de diagnostic selon la revendication 1 ou 2, dans lequel ladite première phase (100) a une durée (T1) comprise entre 1 et 5 minutes, de préférence égale à 3 minutes.

4. Dispositif de diagnostic selon l'une quelconque des revendications 1 à 3, dans lequel ladite deuxième phase (200), ladite troisième phase (300) et ladite quatrième phase (400) ont des durées respectives (T2-T4), chacune comprise entre 3 et 6 minutes, de préférence égale à 5 minutes.

5. Dispositif de diagnostic selon l'une quelconque des revendications 1 à 4, dans lequel ladite unité de traitement et de commande (20) est configurée pour calculer une première aire (A11) et une deuxième aire (A21) sous-tendues par l'enveloppe du premier signal filtré (SF1) au sein d'une première fenêtre temporelle (W11) de la deuxième phase (200) et, respectivement, d'une deuxième fenêtre temporelle (W21) de la quatrième phase (400), une troisième aire (A32) et une quatrième aire (A42) sous-tendues par l'enveloppe du second signal filtré (SF2) au sein d'une troisième fenêtre temporelle (W32) de la deuxième phase (200) et, respectivement, d'une quatrième fenêtre temporelle (W42) de la quatrième phase (400), et un premier indice de fonction endothéliale (EI) défini comme le produit du rapport entre la deuxième aire (A21) et la première aire (A11) par le rapport entre la troisième aire (A32) et la quatrième aire (A42).

6. Dispositif de diagnostic selon la revendication 5, dans lequel lesdites première, deuxième, troisième et quatrième fenêtres temporelles (W11, W21, W32, W42) ont des durées respectives comprises chacune entre 0,5 et 1,5 minute, de préférence égales à 1 minute.

7. Dispositif de diagnostic selon la revendication 5 ou 6, dans lequel ladite deuxième fenêtre temporelle (W21) commence après un délai prédéterminé (D) à compter de la fin de la troisième phase (300), ledit délai (D) étant compris entre 0,5 et 1,5 minute, de préférence égal à 1 minute.

8. Dispositif de diagnostic selon l'une quelconque des revendications 1 à 7, dans lequel ladite unité de traitement et de commande (20) est configurée pour calculer une amplitude moyenne du premier signal filtré (SF1) pendant la deuxième phase (200) et un second indice de fonction endothéliale (RT) correspondant à l'intervalle de temps, au cours de la quatrième phase (400), compris entre l'instant où le premier signal filtré (SF1) atteint son amplitude maximale et l'instant où le premier signal filtré (SF1) atteint ladite amplitude moyenne.

9. Dispositif de diagnostic selon l'une quelconque des revendications 1 à 8, dans lequel l'unité de traitement et de commande (20) est configurée pour sélectionner, à partir du premier signal filtré (SF1) dans la deuxième phase (200) et/ou dans la quatrième phase (400), un nombre prédéterminé de portions de signal correspondant aux formes d'onde d'autant de battements cardiaques aléatoires, de calculer, pour chacun desdits battements cardiaques, une valeur d'au moins un paramètre cardiaque sur la base de sa forme d'onde, et de calculer la valeur moyenne dudit paramètre cardiaque parmi toutes les valeurs desdits battements cardiaques.

10. Dispositif de diagnostic selon l'une quelconque des revendications 1 à 9, dans lequel chacun des capteurs photopléthysmographiques (8, 9) comprend une source de lumière ou de rayonnement infrarouge (8a, 9a) et un récepteur correspondant (8b, 9b), et l'unité de traitement et de commande (20) est configurée pour commander l'unité de mesure (13) de telle sorte que cette dernière, au cours de ladite première phase (100), mesure et vérifie l'amplitude d'un indice de perfusion pour les deux capteurs photopléthysmographiques (8, 9) sur la base des premier et second signaux (S1, S2), et ajuste l'intensité du rayonnement émis par les sources de rayonnement (8a, 9a) afin d'adapter le fonctionnement des capteurs photopléthysmographiques (8, 9) aux caractéristiques du tissu du doigt et à la lumière ambiante.

11. Dispositif de diagnostic selon l'une quelconque des revendications 1 à 10, dans lequel ledit au moins un module de prétraitement (23-25) comprend un filtre passe-bas (23) et au moins un module d'élimination des artefacts (24), ce dernier étant destiné à éliminer les artefacts présents sur les premier et second signaux (S1, S2) dus aux mouvements des premier et second doigts (10, 11) par rapport aux capteurs photopléthysmographiques (8, 9).
